Europäisches Patentamt

(19) European Patent Office

Office européen des brevets

(11) EP 1 605 027 A1

(12) EUROPEAN PATENT APPLICATION
published in accordance with Art. 158(3) EPC

(43) Date of publication:
14.12.2005 Bulletin 2005/50

(51) Int Cl.7: C09K 3/00, A61K 7/00,
A61K 7/42, C09C 1/28,
C09C 3/08

(21) Application number: 04718753.9

(22) Date of filing: 09.03.2004

(86) International application number:
PCT/JP2004/003006

(87) International publication number:
WO 2004/081137 (23.09.2004 Gazette 2004/39)

(84) Designated Contracting States:
AT BE BG CH CY CZ DE DK EE ES FI FR GB GR
HU IE IT LI LU MC NL PL PT RO SE SI SK TR
Designated Extension States:
AL LT LV MK

(30) Priority: 14.03.2003 JP 2003069162

(71) Applicants:
• THE NISSHIN OIL MILLS, LTD.
Tokyo, 104-8285 (JP)
• FUJI SILYSIA CHEMICAL LTD.
Kasugai-shi Aichi 4870013 (JP)
Designated Contracting States:
EE

(72) Inventors:
• MIZUOKA, Takanori,
C/o Nisshin Oillio Group, Ltd
Yokohama-shi, Kanagawa 235-8558 (JP)

• IWAMOTO, Yoshiaki,
C/o Nisshin Oillio Group, Ltd
Yokohama-shi, Kanagawa 235-8558 (JP)
• TAKAGI, Yoshiaki, C/o Nisshin Oillio Group, Ltd
Yokohama-shi, Kanagawa 235-8558 (JP)
• ASHITAKA, Keiji, C/o Fuji Silysia Chemical Ltd
Kasugai-shi, Aichi 487-0013 (JP)
• NOBUHARA, Kazunori,
C/o Fuji Silysia Chemical Ltd
Kasugai-shi, Aichi 487-0013 (JP)

(74) Representative: Curtis, Philip Anthony et al
A.A. Thornton & Co.,
235 High Holborn
London WC1V 7LE (GB)

(54) METHOD FOR REFINING ULTRAVIOLET ABSORBENT, ULTRAVIOLET ABSORBENT AND COSMETIC MATERIAL

(57) The process for purifying an ultraviolet absorber of the present invention is characterized by including an adsorbent treatment step of bringing the ultraviolet absorber into contact with an absorbent. According to the process, an ultraviolet absorber having a reduced odor, an improved hue and less skin irritation can be obtained.

## Description

Technical Field

The present invention relates to a process for purifying ultraviolet absorbers, in particular, to a process for purifying ultraviolet absorbers in which aldehydes, as causative substances of odor, contained in the ultraviolet absorbers are specifically removed. The present invention also relates to an ultraviolet absorber having a reduced odor and an improved hue and cosmetics that contain a purified ultraviolet absorber.

Background Art

To prevent harmful effects on the skin by ultraviolet rays, many cosmetics having ultraviolet preventing effect have been developed. Traditionally, such cosmetics contain an ultraviolet absorber such as a derivative of cinnamic acid, benzophenone, para-aminobenzoic acid or salicylic acid. Of the above described derivatives, derivatives of cinnamic acid or salicylic acid have been often used, from the viewpoint of safety, compatibility with cosmetic base materials and ultraviolet absorptivity. As the cinnamic acid or salicylic acid derivatives, there can be mentioned; ester compounds such as ethyl p-methoxycinnamate; isopropyl p-methoxycinnamate; 2-ethylhexyl p-methoxycinnamate; glyceryl mono-2-ethylhexanoate di-p-methoxycinnamate; octyl salicylate; phenyl salicylate; homomenthyl salicylate; dipropyleneglycol salicylate; ethyleneglycol salicylate; myristyl salicylate; and methyl salicylate.

Generally, the above described ultraviolet absorbers are poor in long-term stability, and thus they can be oxidized and deteriorate with time and sometimes give an odor or color. Thus, the cosmetics contain such ultraviolet absorbers together with a stabilizer, such as butylhydroxy toluene (BHT) or tocopherol as an antioxidant. However, ultraviolet absorbers composed of a compound having an aromatic structure often contain substances having a characteristic odor or skin irritation or a coloring material from the outset. Even if an antioxidant is added to such ultraviolet absorbers, the substances or coloring material remains in the ultraviolet absorbers; as a result, when using cosmetics or the like that contain an sufficient amount of ultraviolet absorber to give ultraviolet preventing effect, one can sometimes feel a characteristic odor or skin irritation or find color in them. Thus, an ultraviolet absorber having a reduced odor, color and skin irritation have been demanded.

As one example of processes for reducing an odor of ultraviolet absorbers, Japanese Patent Application Laid-Open No. 1995-89835 discloses a process in which odor substances contained in an ultraviolet absorber are removed by physical adsorption, specifically by bringing the ultraviolet absorber having been diluted with a solvent, such as hexane, into contact with an adsorbent such as silica gel. By the process disclosed in the above patent application, however, butylhydroxy toluene or tocopherol, which has been added as an antioxidant to the ultraviolet absorber, is also adsorbed together with the odor substances by the adsorbent; thus, the process possesses a problem of being unable to suppress the deterioration with time of the ultraviolet absorption or the formulated cosmetics.

Further, the process disclosed in the above described patent application requires a large amount of solvent and adsorbent, which is problematic in terms of safety or environments of the operation as well as cost, when it is used in industrial production; thus, the process is not practical.

Accordingly, the object of the present invention is to provide a process for purifying an ultraviolet absorber, that is excellent in industrial productivity, an ultraviolet absorber obtained by the above purifying process which has a reduced odor, an improved hue and less skin irritation, and cosmetics using the ultraviolet absorber.

Disclosure of the Invention

The inventors of the present invention carried out an in-depth study to accomplish the object. As a result, they have found that an ultraviolet absorber having a reduced odor and an improved hue can be obtained by bringing an ultraviolet absorber into contact with an adsorbent. And they have finally accomplished the present invention.

The present invention has been accomplished based on the above finding. According to the present invention, there is provided a process for purifying an ultraviolet absorber, characterized by including an adsorbent treatment step of bringing an ultraviolet absorber into contact with an adsorbent.

According to the above described process for producing an ultraviolet absorber, aldehydes contained in an ultraviolet absorber can be selectively removed, and thus an ultraviolet absorber having a reduced odor and an improved hue can be obtained.

The above described adsorbent is preferably an amino-modified silica gel obtained by allowing silica gel to react with a silane coupling agent having a primary amine group to modify the surface of the silica gel.

The above described adsorbent is preferably such that the amount of the primary amine group supported on the adsorbent is 0.4 to 1.5 µmole per mg of silica gel.

The above described ultraviolet absorber preferably contains at least one kind of ester compound that is composed of an aromatic fatty acid and a monohydric- or polyhydric-alcohol.

The above described ultraviolet absorber preferably contains at least one kind of ester compound that is composed of methoxycinnamic acid and a monohydric- or polyhydric-alcohol.

The above described process for purifying an ultraviolet absorber preferably includes a deodorizing treatment step after the above described adsorbent treatment step.

The present invention also provides an ultraviolet absorber having been purified by the above described process for purifying an ultraviolet absorber.

The present invention also provides a cosmetic that contains the above described ultraviolet absorber.

Best Mode for Carrying Out the Invention

A process for purifying ultraviolet absorbers according to the present invention is described in detail below.

The term "ultraviolet absorber" as used herein means an ultraviolet absorber containing, as a main ingredient, at least one kind of ester compound that is composed of an aromatic fatty acid and a monohydric- or polyhydric-alcohol. As such ester compound, there can be mentioned; ethyl p-methoxycinnamate; isopropyl p-methoxycinnamate; 2-ethylhexyl p-methoxycinnamate; glyceryl mono-2-ethylhexanoate di-p-methoxycinnamate; octyl salicylate; phenyl salicylate; homomenthyl salicylate; dipropyleneglycol salicylate; ethyleneglycol salicylate; myristyl salicylate; methyl salicylate; and the mixture thereof with other general-purpose ester compounds. An ultraviolet absorber containing, as a main ingredient, at least one kind of ester compound that is composed of methoxycinnamic acid and a monohydric- or polyhydric-alcohol is particularly preferable as the ultraviolet absorbers which the process for purifying an ultraviolet absorber of the present invention is intended for. Such ultraviolet absorber is preferably used in cosmetics, from the viewpoint of safety and ultraviolet absorptivity.

The process for purifying an ultraviolet absorber according to the present invention includes an adsorbent treatment step of bringing an ultraviolet absorber into contact with an adsorbent.

The adsorbent treatment may be carried out by first dissolving an ultraviolet absorber in a solvent and then bringing the ultraviolet absorber dissolved in the solvent into contact with an adsorbent or by bringing an ultraviolet absorber directly into contact with an adsorbent while avoiding the process of dissolving the ultraviolet absorber in a solvent. When the process employs a solvent, as the solvents applicable, there can be mentioned; n-hexane, n-heptane, petroleum ether, ethyl acetate, isobutyl acetate, methyl ethyl ketone, ethyl alcohol, isopropyl alcohol, and the mixtures thereof.

In the present invention, however, it is preferable not to use a solvent, from the viewpoint of industrial productivity (production cost), influence on the environment, reduction of the number of steps, and safety during the production. In other words, it is preferable to bring an ultraviolet absorber directly into contact with an adsorbent.

The adsorbent used in the present invention is for adsorbing odor substances contained in an ultraviolet absorber. As such adsorbents, there can be mentioned; synthetic adsorbents containing an oxide (or hydroxide) of Mg, Al or Si, as a constituent. Concrete examples include: silica gel; activated clay; activated alumina/magnesia anhydride; and active alumina/magnesia hydrate. Either one of these adsorbents alone or two or more in a properly mixed form can be used.

The particle diameter of the adsorbent used is preferably 0.1 to 3 mm, more preferably 0.1 to 0.5 mm. If the particle diameter is within the above described range, a sufficient adsorbent treatment speed can be obtained, and moreover, the adsorbent can be easily separated from the ultraviolet absorber after the adsorbent treatment.

As the adsorbents applicable to the process for purifying ultraviolet absorbers of the present invention, there can be mentioned; silica gel with a primary amine group supported thereon. Such silica gel is preferably used in the process for purifying ultraviolet absorbers of the present invention.

A primary amine group reacts specifically with aldehydes to form orange to red Schiff's salts, as shown in the reaction formula below. Since a primary amine group and an aldehyde chemically bond with each other, the aldehyde never leaves from the adsorbent. The adsorbent reacting specifically with aldehydes can selectively remove aldehydes contained in ultraviolet absorbers, even if the concentration of the aldehydes is very low.

$$\text{Silica-NH}_2 + \text{R-CHO} \rightarrow \text{Silica-N} = \text{CHR} + \text{H}_2\text{O}$$

As the adsorbent used in the process for purifying ultraviolet absorbers of the present invention, amino-modified silica gel is preferable which is obtained by allowing silica gel to react with a silane coupling agent having a primary amine group to modify the surface of the silica gel. Such amino-modified silica gel has the advantage that aldehydes as described above do not leave from it, because the primary amine group is not liberated from the silica gel easily due to the chemical bond of the silanol group to the surface of the silica gel, and therefore it chemically bonds with aldehydes.

Processes for preparing amino-modified silica gel include: for example, not limited to, a process which includes the steps of: making silica gel into a slurry with an appropriate solvent; adding a silane coupling agent having a primary amine group; allowing the silica gel and the silane coupling agent to react with each other at ordinary temperature to 120°C for 2 to 24 hours; if necessary, cleaning and solid-liquid separating the mixture; and drying the same.

The silica gel used in the process for purifying ultraviolet absorbers of the present invention preferably has pore diameter of 5 nm to 40 nm and more preferably 6 to 15 nm, considering its reactivity with a silane coupling agent and its steric effect with aldehydes. Further, the silica gel used in the process for purifying ultraviolet absorbers of the present invention preferably reacts with a silane coupling agent on a large specific surface area. Specifically, the specific surface area is preferably 100 $m^2/g$ to 800 $m^2/g$.

As the silane coupling agents having a primary amine groups, there can be mentioned; 4-aminobutyltriethoxysilane, 2-aminoethylaminomethylbenzyloxydimethylsilane, (aminoethylaminomethyl)phenethyltrimethoxysilane, N-(2-aminoethyl)-aminopropyltrimethoxysilane, N-(6-aminohexyl)-aminopropyltrimethoxysilane, 3-(m-aminophenoxy) propyltrimethoxysilane, m-aminophenyltrimethoxysilane, p-aminophenyltrimethoxysilane, 3-aminopropyldiisopropylethoxysilane, 3-aminopropyldimethylethoxysilane, 3-aminopropylmethyldiethoxysilane, aminopropylsilanetriol, 3-aminopropyltriethoxysilane, 3-aminopropyltrimethoxysilane, 3-aminopropyltris(trimethylsiloxy)silane, 3-aminopropyltriethoxysilane, 1,3-bis(3-aminopropyl)tetramethyldisiloxane, N-(2-aminoethyl)-aminopropylmethyldimethoxysilane, 3-(1-aminopropoxy)-3,3-dimethyl-1-propenyltrimethoxysilane and 3-aminopropyltris(methoxyethoxyethoxy)silane.

The amount of primary amine group supported on silica gel is preferably, not limited to, 0.4 to 1.5 μmole per mg of silica gel, more preferably 0.5 to 1.2 μmole per mg of silica gel, considering the amount of aldehydes adsorbed by the silica gel. If the amount of the primary amine group supported by silica gel is less than 0.4 μmole/mg, the amount of the adsorbent required is increased, which can make the process uneconomical.

In the process for purifying ultraviolet absorbers of the present invention, aldehydes are mainly removed in the adsorbent treatment step. The main causative substances of the odor characteristic to ultraviolet absorbers that contain an ester compound of aromatic fatty acid are aldehydes. And such aldehydes are selectively removed by the process for purifying ultraviolet absorbers of the present invention, whereby ultraviolet absorbers having a reduced odor and an improved hue can be obtained efficiently.

The amount of the adsorbent used is preferably 0.5 to 10 parts by mass per 100 parts by mass of ultraviolet absorber and preferably 1 to 5 parts by mass. If the amount of the adsorbent used is in the range of 0.5 to 10 parts by mass per 100 parts by mass of the ultraviolet absorber, sufficient decoloring and deodorizing effects can be obtained while maintaining the industrial productivity and economy.

The adsorbent treatment step of treating an ultraviolet absorber with an adsorbent by bringing the two into contact with each other can be embodied by various processes. Examples of the processes which embody the step include: a process in which an ultraviolet absorber and an adsorbent are mixed by stirring; a process in which an ultraviolet absorber is passed through an adsorbent having been packed into a vessel such as a column; and a process in which an ultraviolet absorber is passed through a moving bed of adsorbent.

In the process for purifying ultraviolet absorbers according to the present invention, it is preferable, from the viewpoint of industrial productivity (production cost), influence on the environment, reduction of the number of steps, and safety during the production, not to use a solvent, as described above.

Processes for mixing an ultraviolet absorber and an adsorbent by stirring without using a solvent include: for example, a process in which an adsorbent is directly added to an ultraviolet absorber and the mixture is heated with stirring preferably at ordinary temperature to 120°C, more preferably at ordinary temperature to 100°C, and most preferably ordinary temperature to 80°C. The temperature lower than ordinary temperature causes the viscosity of the ultraviolet absorber to be increased, which may result in poor workability when, for example, carrying out filtration. On the other hand, the temperature higher than 120°C causes the ultraviolet absorber to deteriorate by heating. In addition to the mixing operation with stirring, operations for improving the dispersion efficiency, such as vibrating the vessel or using ultrasonic, can also be employed depending on the situation.

In the process for purifying ultraviolet absorbers of the present invention, deodorizing treatment may be carried out after the adsorbent treatment step. Adding a deodorizing treatment step makes it possible to remove odor substances which cannot be removed in the adsorbent treatment step, thereby producing a much highly purified ultraviolet absorber. Particularly when using, as an adsorbent, amino-modified silica gel, which is obtained by allowing silica gel to react with a primary amine group-containing silane coupling agent, aldehydes are specifically adsorbed and removed by the amino-modified silica gel, but other odor substances such as alcohols are not removed, and therefore it is preferable to provide a deodorizing treatment step after the adsorbent treatment step.

The process for carrying out the deodorizing treatment step in the process for purifying ultraviolet absorbers of the present invention is not limited to any specific one, and the deodorizing treatment step can be carried out by any one of the processes commonly used in deodorization of synthetic esters, fat and oil materials or tocopherol.

Processes for carrying out the deodorizing treatment step include: for example, a process in which an ultraviolet absorber is heated under reduced pressure while blowing nitrogen gas or water vapor over the ultraviolet absorber. The degree of vacuum is preferably 4000 Pa or lower, and more preferably 1000 Pa or lower. If the degree of vacuum is higher than 4000 Pa, the deodorizing treatment has to be carried out at higher temperatures, and heating at such high temperatures may cause the ultraviolet absorber to deteriorate. The heating temperature is preferably 30 to 250°C and more preferably 80 to 200°C. If the temperature is lower than 30°C, the odor cannot be fully removed, whereas if the temperature is higher than 250°C, the ultraviolet absorber may be decomposed. The deodorizing treatment time is preferably 30 minutes to 20 hours and more preferably 1 hour to 10 hours. If the treatment time is longer than 20 hours, the ultraviolet absorber may deteriorate due to the heat.

As described above, in the process of the present invention, it is preferable to carry out the deodorizing treatment step after the adsorbent treatment step. If the deodorizing treatment step is carried out before the adsorbent treatment step, not only another odor substances may be generated but also the hue may be worsened by heating, which may make it impossible to fully purify the ultraviolet absorber in the subsequent adsorbent treatment step.

Further, in the process for purifying ultraviolet absorbers according to the present invention, it is preferable to provide a step of removing the adsorbent by filtration. If an ultraviolet absorber undergoes deodorizing treatment with an adsorbent remaining therein, substances having a distinctive odor may be generated by heating at high temperatures, and the odor substances are unlikely to be removed even by the subsequent filtration of the adsorbent.

Processes for removing the adsorbent include: besides filtration, centrifugation and sedimentation.

The ultraviolet absorber according to the present invention is an ultraviolet absorber having been purified by the process for purifying ultraviolet absorbers of the present invention. The ultraviolet absorber according to the present invention has a reduced odor and an excellent hue, compared with unpurified ultraviolet absorbers. Further, the ultraviolet absorber according to the present invention contains a reduced amount of skin irritating substances, and thus its safety has been improved.

The ultraviolet absorber according to the present invention can be used in all the products required to have ultraviolet absorbing effects. As the products, there con be mentioned; sunscreen cosmetics, suntan cosmetics, hand creams, lipsticks and foundations.

The followings are detailed description about the cosmetics according to the present invention. The cosmetics according to the present invention contain an ultraviolet absorber according to the present invention. The ingredients for the cosmetics other than the ultraviolet absorber according to the present invention are those commonly used as ingredients for ordinary cosmetics and are not limited to any specific ones. As the ingredients contained in the cosmetics according to the present invention, there can be mentioned; solid, semisolid or liquid oil materials (e.g. natural animal or vegetable fats and oils, semisynthetic fats and oils, hydrocarbon oils, higher fatty acids, higher alcohols, ester oils, silicone oils and fluorine oils); water; alcohols (e.g. lower alcohols, sugar alcohols, sterols and the like); water-soluble polymers (e.g. polymers derived from vegetables such as gum arabic and gum tragacanth, polymers derived from microorganisms such as xantan gum, dextran, polymers derived from starches such as carboxymethyl starch and polymers derived from cellulose such as carboxymethyl cellulose sodium); surfactants (e.g. various types of anionic, cationic, nonionic or amphoteric surfactants); oil-soluble gelling agents (e.g. metallic soap, dextrin fatty acid ester and sucrose fatty acid ester); powders (e.g. inorganic powders such as titanium oxide, magnesium carbide, mica or hydroxyapatite, and organic powders such as polyamide powder); color pigments; pearl pigments; humectants; antiseptics; pH adjustors; chelating agents; refreshing materials; anti-inflammatory agents; skin care ingredients (e.g. whitening ingredients, cell activating ingredients and blood circulation improver); and vitamins.

The amount of the ultraviolet absorber contained in the cosmetics of the present invention is not limited to any specific one, as long as it allows a desired ultraviolet absorbing effect to be produced. Usually, preferably the amount is 0.5 to 20% by mass per 100% by mass of the cosmetics and more preferably 2 to 10% by mass.

Example

The present invention will be described in more detail by way of examples, below. Such examples, however, are not to be construed as limiting in any way the scope of the present invention.

Example 1

Amino-modified silica gel as an adsorbent was obtained by treating "MB 5D" (spherical silica gel with a pore diameter of 10 nm and a specific surface area of 250 m$^2$/g), trade name, manufactured by FUJI SILYSIA CHEMICAL LTD. with a silane coupling agent having a primary amine group to modify the surface of the silica gel. The amount of the primary amine group supported by the resultant adsorbent was 0.7 μmole per mg of silica gel.

Then, adsorbent treatment was carried out in such a manner as to first add 1000 g of 2-ethylhexyl p-methoxycinnamate, a commercially available ultraviolet absorber, to a vessel equipped with a stirrer, then add 10 g of the above described adsorbent, and stir the mixture at 80°C for 1 hour. Then, the adsorbent was filtered to obtain a purified oil that contains an ultraviolet absorber from which the adsorbent had been removed.

The obtained purified oil was fed into a three-neck flask equipped with a nitrogen gas blowing pipe and a thermometer, and deodorizing treatment was carried out by heating the purified oil under reduced pressure, at 100 to 140°C and 200 to 800 Pa, for 3 hours while blowing nitrogen gas into the flask to obtain a purified ultraviolet absorber. Evaluations were performed for the obtained ultraviolet absorber shown below. The results are shown in Table 1. Evaluations were also made for the commercially available untreated ultraviolet absorbers in the same manner as in example 1. The results are shown, as those of comparative example 1, in Table 1.

<Evaluations for ultraviolet absorbers>

(Odor evaluation for ultraviolet absorbers)

Sensory evaluation of odor was performed for the ultraviolet absorbers. The intensity of overall odor and that of irritating odor were evaluated using one of 10 ranks: 1, no odor; ...; 10, very intense odor and scored.

(Hue evaluation for ultraviolet absorbers)

Hazen color (APHA) was measured using Lovibond Nessleriser 2150.

(Odor evaluation for ultraviolet absorbers after a time has elapsed)

100 ml of each ultraviolet absorber was filled into two different 200-ml sample bottles, and one bottle was stored in the dark (at 50°C) and the other in the light (at 20°C, 1000 lux) for 3 weeks. The intensity of overall odor and that of irritating odor were evaluated using one of 10 ranks: 1, no odor; ...; 10, very intense odor and scored.

Example 2

An ultraviolet absorber was obtained in the same manner as in example 1, provided that in the deodorizing treatment, instead of nitrogen gas, water vapor was blown into the flask. Evaluations were performed for the obtained ultraviolet absorber in the same manner as in example 1. The results are shown in Table 1.

Example 3

An ultraviolet absorber was obtained in the same manner as in example 1, provided that the amount of the adsorbent used was 100 g. Evaluations were performed for the obtained ultraviolet absorber in the same manner as in example 1. The results are shown in Table 1.

Example 4

100 g of adsorbent, the same type as used in example 1, was filled into a glass column 30 mm in diameter and a solution of 1000 g of commercially available ultraviolet absorber, the same type as used in example 1, in 2000 g of n-hexane as a solvent was passed through the adsorbent. Then, the solvent was removed from the discharge solution to obtain a purified oil containing the ultraviolet absorber. The same deodorizing treatment as in example 1 was performed for the obtained purified oil to obtain an ultraviolet absorber. Evaluations were performed for the obtained ultraviolet absorber in the same manner as in example 1. The results are shown in Table 1.

Comparative example 2

Adsorbent treatment was carried out in such a manner as to first add 1000 g of 2-ethylhexyl p-methoxycinnamate, a commercially available ultraviolet absorber, to a vessel equipped with a stirrer, then add 10 g of adsorbent, the same type as used in example 1, and stir the mixture at 80°C for 1 hour. Then, the adsorbent was filtered to obtain a purified oil that contains an ultraviolet absorber from which the adsorbent had been removed. The purified oil thus obtained was used as an ultraviolet absorber, and evaluations were performed for the obtained ultraviolet absorber in the same manner as in example 1. The results are shown in Table 1.

Comparative example 3

1000 g of 2-ethylhexyl p-methoxycinnamate, a commercially available ultraviolet absorber, was fed into a three-neck flask equipped with a nitrogen gas blowing pipe and a thermometer, and deodorizing treatment was carried out by heating the ultraviolet absorber under reduced pressure, at 100 to 140°C and 200 to 800 Pa, for 3 hours while blowing nitrogen gas into the flask to obtain a purified ultraviolet absorber. Evaluations were performed for the obtained ultraviolet absorber in the same manner as in example 1. The results are shown in Table 1.

Comparative example 4

1000 g of 2-ethylhexyl p-methoxycinnamate, a commercially available ultraviolet absorber, was fed into a three-neck flask equipped with a nitrogen gas blowing pipe and a thermometer, and deodorizing treatment was carried out by heating the ultraviolet absorber under reduced pressure, at 100 to 140°C and 200 to 800 Pa, for 3 hours while blowing nitrogen gas into the flask. Then, adsorbent treatment was carried out in such a manner as to first add 1000 g of the ultraviolet absorber having undergone deodorizing treatment to a vessel equipped with a stirrer, then add 10 g of adsorbent, the same type as used in example 1, and stir the mixture at 80°C for 1 hour. Then, the adsorbent was filtered to obtain a purified oil that contains an ultraviolet absorber from which the adsorbent had been removed. Evaluations were performed for the obtained purified oil, as an ultraviolet absorber, in the same manner as in example 1. The results are shown in Table 1.

Comparative example 5

An ultraviolet absorber was obtained in the same manner as in example 1, provided that activated clay was used as an adsorbent. Evaluations were performed for the obtained ultraviolet absorber in the same manner as in example 1. The results are shown in Table 1.

Comparative example 6

An ultraviolet absorber was obtained in the same manner as in example 1, provided that silica gel ("MB 5D", trade name, manufactured by FUJI SILYSIA CHEMICAL LTD.) not having undergone amino modification was used as an adsorbent. Evaluations were performed for the obtained ultraviolet absorber in the same manner as in example 1. The results are shown in Table 1.

Comparative example 7

An ultraviolet absorber was obtained in the same manner as in example 1, provided that activated alumina/magnesia anhydride was used as an adsorbent. Evaluations were performed for the obtained ultraviolet absorber in the same manner as in example 1. The results are shown in Table 1.

Comparative example 8

An ultraviolet absorber was obtained in the same manner as in example 1, provided that activated silica/alumina hydrate was used as an adsorbent. Evaluations were performed for the obtained ultraviolet absorber in the same manner as in example 1. The results are shown in Table 1.

Comparative example 9

Adsorbent treatment was carried out in such a manner that firstly 1000 g of 2-ethylhexyl p-methoxycinnamate, as a commercially available ultraviolet absorber, was added to a vessel equipped with a stirrer, 1000 g of hexane was added as a solvent, then 10 g of activated alumina/magnesia anhydride was added as an adsorbent, and the mixture was stirred at 25°C for 1 hour. Then, the adsorbent was filtered and the solvent was removed from the filtrate to obtain a purified oil that contains the ultraviolet absorber. Then the obtained purified oil was subjected to deodorizing treatment in the same manner as in example 1 to obtain an ultraviolet absorber. Evaluations were performed for the obtained ultraviolet absorber in the same manner as in example 1. The results are shown in Table 1.

Table 1

| | Evaluation for ultraviolet absorber | | Odor evaluation for ultraviolet absorber after a time has elapsed | |
|---|---|---|---|---|
| | Odor | Hue | In the dark | In the light |
| Example 1 | 1 | 10 | 2 | 1 |
| Example 2 | 1 | 10 | 2 | 1 |
| Example 3 | 1 | 10 | 2 | 1 |
| Example 4 | 1 | 10 | 2 | 1 |
| Comparative example 1 | 10 | 40 | 5 | 4 |
| Comparative example 2 | 6 | 10 | 5 | 4 |
| Comparative example 3 | 7 | 40 | 4 | 4 |
| Comparative example 4 | 4 | 10 | 4 | 3 |
| Comparative example 5 | 6 | 20 | 3 | 3 |
| Comparative example 6 | 5 | 20 | 3 | 2 |
| Comparative example 7 | 5 | 10 | 3 | 2 |
| Comparative example 8 | 5 | 20 | 3 | 2 |
| Comparative example 9 | 1 | 10 | 2 | 3 |

As is apparent from Table 1, the ultraviolet absorbers of examples 1 to 4 were ranked high in both the odor and hue evaluations, and moreover, they underwent only a slight change in odor after a time had elapsed. Thus, it is clear that the ultraviolet absorbers of examples 1 to 4 were vastly superior to the unpurified ones. On the other hand, the ultraviolet absorbers of comparative examples 1 to 9 were ranked low compared with those of examples 1 to 4, though their odor and hue were improved compared with the unpurified ultraviolet absorbers.

Advantage of the Invention

According to the present invention, a process for purifying ultraviolet absorbers can be provided which can improve the odor and hue of ultraviolet absorbers and excels in industrial productivity. The use of the purifying process makes it possible to provide ultraviolet absorbers having a reduced odor and an improved hue and excelling in safety. The use of the ultraviolet absorbers, in turn, makes it possible to provide cosmetics having a reduced characteristic odor and less skin irritation and excelling in safety.

**Claims**

1. A process for purifying an ultraviolet absorber, comprising an adsorbent treatment step of bringing the ultraviolet absorber into contact with the adsorbent.

2. The process for purifying an ultraviolet absorber according to claim 1, wherein the adsorbent is amino-modified silica gel obtained by allowing silica gel to react with a silane coupling agent having a primary amine group to modify the surface of the silica gel.

3. The process for purifying an ultraviolet absorber according to claim 1 or 2, wherein the amount of the primary amine group supported on the adsorbent is 0.4 to 1.5 $\mu$mole per mg of silica gel.

4. The process for purifying an ultraviolet absorber according to any one of claims 1 to 3, wherein the ultraviolet absorber comprises at least one kind of ester compound that is composed of an aromatic fatty acid and a monohydric- or polyhydric-alcohol.

5. The process for purifying an ultraviolet absorber according to any one of claims 1 to 4, wherein the ultraviolet absorber comprises at least one kind of ester compound that is composed of methoxycinnamic acid and a monohydric- or polyhydric-alcohol.

6. The process for purifying an ultraviolet absorber according to any one of claims 1 to 5, further comprising a deodorizing treatment step after the adsorbent treatment step.

7. An ultraviolet absorber, purified by the process for purifying an ultraviolet absorber according to any one of claims 1 to 6.

8. A cosmetic containing an ultraviolet absorber according to claim 7.

| INTERNATIONAL SEARCH REPORT | International application No. |
|---|---|
| | PCT/JP2004/003006 |

A. CLASSIFICATION OF SUBJECT MATTER
Int.Cl⁷ C09K3/00, A61K7/00, A61K7/42, C09C1/28, C09C3/08

According to International Patent Classification (IPC) or to both national classification and IPC

B. FIELDS SEARCHED

Minimum documentation searched (classification system followed by classification symbols)
Int.Cl⁷ C09K3/00, A61K7/00-7/50, C09C1/28-1/30, C09C3/08,
B01J20/10-20/18

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 7-89835 A (Kuroda Japan Kabushiki Kaisha),<br>04 April, 1995 (04.04.95),<br>Claim 1<br>(Family: none) | 1,4,5,7,8<br>2,3,6 |
| A | JP 8-257346 A (Nippon Soken, Inc.),<br>08 October, 1996 (08.10.96),<br>(Family: none) | 1-8 |
| A | JP 6-7634 A (Nippondenso Co., Ltd.),<br>18 January, 1994 (18.01.94),<br>(Family: none) | 1-8 |

☐ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 20 May, 2004 (20.05.04) | 15 June, 2004 (15.06.04) |

| Name and mailing address of the ISA/ | Authorized officer |
|---|---|
| Japanese Patent Office | |
| Facsimile No. | Telephone No. |

Form PCT/ISA/210 (second sheet) (January 2004)